# EUROPEAN PATENT APPLICATION

(11) **EP 1 496 358 A1**
(43) Date of publication of application: **12.01.2005**
(21) Application number: 03745935.1
(22) Date of filing: 07.04.2003
(51) Int. Cl.: G01N 33/50, A61B 5/00, G06F 17/60

(54) **METHOD OF ESTIMATING RISK OF DENTAL DECAY, APPARATUS OF ESTIMATING RISK OF DENTAL DECAY, SYSTEM OF ESTIMATING RISK OF DENTAL DECAY AND PROGRAM OF ESTIMATING RISK OF DENTAL DECAY**

(30) Priority: 10.04.2002 JP 2002107712; 05.08.2002 JP 2002227319
(71) Applicant: Horiba, Ltd., Kyoto-shi, Kyoto 601-8510 (JP)
(72) Inventor: NOMURA, Satoshi, c/o Horiba, Ltd., Kyoto-shi, Kyoto 601-8510 (JP); HONJO, Mitsuru, c/o Horiba, Ltd., Kyoto-shi, Kyoto 601-8510 (JP); TAGAMI, Junji, c/o Tokyo Medical and Dental, Tokyo 113-8549 (JP); KITASAKO, Yuichi, c/o Tokyo Medical and Dental, Tokyo 113-8549 (JP)
(74) Representative: Müller - Hoffmann & Partner
(86) International application number: PCT/JP2003/004380
(87) International publication number: WO 2003/085398

(57) **Abstract**

The present invention provides a method of evaluating the risk of possible caries and the device, system, and program of the same, which is useful for the subjects to keep their mouth conditions best by measuring the chemical properties of saliva samples collected from the mouths of subjects to allow for easy and sanitary evaluation of the risk of possible caries in a short time and to help the subjects accurately recognize their risk of possible caries and closely consult their dentist.

In the present invention, saliva from the mouth of subject is collected, a given ratio of acid is added to the saliva, and the chemical property of the saliva is measured to evaluate the risk of possible caries base on the measured value.

## Description

### Technical Field

The present invention relates to a method of accurately evaluating the risk of possible dental caries by measuring the chemical properties of samples collected from subjects such as saliva and the device, system, and program of the same and in particular to an evaluation of the risk of possible dental caries using saliva samples on a chemical property measuring device capable of micro measurement and noninvasive measurement,

### Background Art

Conventionally, dentists have visually examined patients' mouths to check for any the dental caries in diagnosing their dental health. This type of dental health examination is, for example, done at every six months. Then the examinee's mouth condition is kept better by easy treatment because every dental caries is detected before it becomes to need complex treatment.

On the other hand, this method has a problem in that a variance among individuals can not be avoided in determining the resistivity of their mouths to possible caries, namely the their risk of possible dental caries. For that reason, it is sufficient for some patients to undergo mouth health examination at a longer interval, while it is required for other patients to do so at a shorter interval.

Moreover, in the case where a patient undergoes the examination by his/her own dentist, the dentist can estimate the risk of possible dental caries to some extent from his/her examination history, though it is difficult for the dentist, even having many experiences, to accurately determine such a risk in new patients only by visually examining their mouths. Accordingly, in some cases, the dentist requests the patient to come again for re-examination in a short time, imposing a burden on the patient.

Furthermore, the patient, who requests the dentist to examine his/her mouth, need to undergo the correct treatment by him, though it is difficult for him/her to well understand the need for treatment that the dentist determines to be indispensable. In this context, some patients discontinue their treatment at the same time they obtain relief from pain even in midcourse of treatment and they tend to postpone the resumption of treatment if being requested by the dentist to come again because they can not easily detect any caries at the first stage in a mirror.

Although the dentists suggest their patients the importance of keeping the patients' mouth conditions better, it is difficult for them to give an advice to the patients on treating any invisible caries or removing the risk of possible dental caries.

To address this situation, the caries risk test is commonly used using saliva samples collected from the patients' mouths for determining whether their mouth conditions are subject to caries.

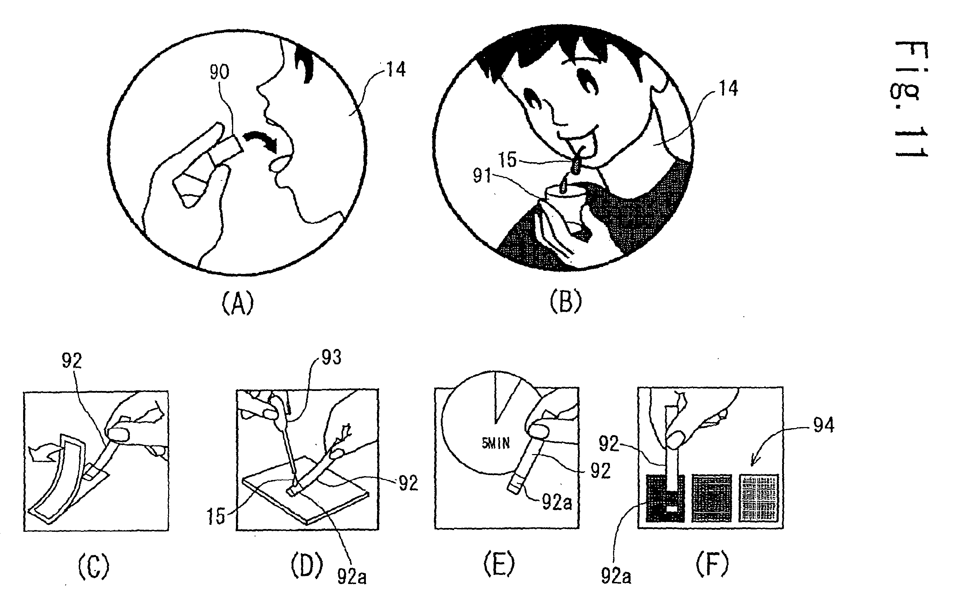

FIG. 11 is a view explaining an example of a conventional caries risk test technique. First, as show in FIG. 11A, to collect a measurable amount of saliva, the subject 14 bites a paraffin pellet 90 and the like for inducing the secretion of saliva.

Second, as show in FIG. 11B, the secreted saliva sample 15 is collected into a container 91, for example a beaker. Then, as shown in FIG. 11C, individually packed test strip 92, to which a drug for measuring their pH buffer capacity has been applied, is taken out from the pack.

FIG. 11D is a view showing a procedure involving steps for sucking out the collected saliva sample 15 using a pipette 93 and the like and dropping it on the pH buffer capacity measurement point 92a with the drug on the test strip 92 until all of them are wetted with the saliva sample 15. As shown in FIG. 11E, after being left for five minutes, the color of the pH buffer capacity measurement point 92a on the test strip 92 wetted with the saliva sample 15 changes.

Finally, as shown in FIG. 11F, the changed color of the pH buffer capacity measurement point 92a is compared with those displayed in a prepared color chart 94 to evaluate the risk of possible dental caries based on the collected saliva sample 15.

However, the conventional caries risk test technique has a problem in that the subject 14 or a laboratory technician may come contact with the saliva sample at a high provability because it involves the steps for collecting a large amount of saliva sample 15 into the container 91 and dripping it on the strips 92 using the pipette 93 and the like, leading to insanitation. Moreover, to collect a measurable amount of saliva sample 15 into the container 91, the subject 14 needs to bite the paraffin pallet 90, taking on a burden.

Moreover, the evaluation technique using the strip 92 does not allow the risk of possible caries to be evaluated easily in a short time because it requires five minutes. Furthermore, it has another problem in that the used strip 92 can not be reused, leading to a increase in cost for evaluating the risk of possible caries.

In the case where an attempt is made to determine whether any caries have developed based on the change in the strip's color after a given time has passed, the timing of comparison with the color chart 94 is an important factor, which imposes time constraints on the measurement procedure. In addition, change in color of the strip 92 may vary among the individual strips, which disturbs the determination of the risk of possible caries in some cases.

This means that the conventional method of evaluating the risk of possible caries is prevented from convenient application because it is not only unsanitary but also requires extensive labor and time on both sides of the laboratory technician and the patient. For that reason, almost few patients are checked for any risk of possible caries at a dental clinic and they can not well recognize their risk of possible caries.

In the case where a patient visits to another dentist due to, for example the change,of his/her house, the dentist should create the new medical record of the patient and recognize the patient's mouth condition while treating a dental disease because he/her can not know his/her history of disease.

The objective of the present invention, in which the solutions for the problems mentioned above have been considered, is to provide a method of evaluating the risk of possible caries and the device, system, and program of the same, which is useful for the subjects to keep their mouth conditions best by measuring the chemical properties of saliva samples collected from the mouths of subjects to allow for easy and sanitary evaluation of the risk of possible caries in a short time and to help the subjects accurately recognize their risk of possible carles and closely consult their dentist.

### Disclosure of the Invention

To attain the objective mentioned above, the method of evaluating the risk of caries in the first mode of the present invention has such a characteristic that saliva is collected from the mouth of a subject, a given ratio of acid is added to the saliva, and the chemical property of the saliva is measured to evaluate the risk of possible caries base on the measured value.

The method of evaluating the risk of possible caries in the second mode of the present invention has such a characteristic that the saliva is collected from the mouth of the subject, the chemical property of the saliva is measured using a chemical property measuring device, a given ratio of acid is added to the saliva, and the chemical property of saliva is measured again to evaluate the risk of possible caries based on the difference between the measured value of chemical property of saliva before adding acid and that after adding acid.

Thus, the method of evaluating the risk of possible caries of the present invention allows for the measurement of the acid buffer capacity of the subject's saliva, achieving the correct evaluation of the risk of possible caries. This means that the saliva can be used to evaluate the risk of possible caries because the saliva's buffer capacity regulates the chemical properties of sailva itself if appropriate to prevent the mouth condition from being acidic, in which caries tend to easily develop.

Moreover, the method of evaluating the risk of possible caries using the chemical property measuring device can be repeatedly used, reducing a running cost for one evaluation.

For that reason, if a dentist or a patient himself/herself uses the method of the risk of possible caries of the present invention, the mouth condition could be measured conveniently to obtain an objective indication for treatment. In addition, the dentist could give an advice to the patient on how to prevent caries from developing and treat him/her correctly to teach the patient the method for mouth care depending on his/her risk of possible caries. The patient would tent to adhere to the dentist's advice based on the measured data without anxiety, relieving a pain involved with treatment.

A more accurate quantity of acid with a given ratio to saliva could be mixed in the saliva by determining the quantity of the saliva to be collected and immersing an absorbing piece, which absorbs a given ratio of acid, in the saliva. When, in particular, a minute quantity of acid is mixed in the saliva, the given ratio of acid could be easily mixed in the saliva samples, resulting in easy measurement by regulating the quantity of saliva to be mixed with the quantity of acid to be absorbed by the absorbing piece determined. The use of the absorbing piece makes the mixture of a given quantity of acid into the saliva very easy, facilitating the evaluation of the risk of possible caries.

The device of evaluating the risk of possible caries in the third mode of the present invention has such a characteristic that it is composed of a container for storing the saliva collected from the mouth of the subject, a sensor surface, which comes in contact with the saliva to measure the chemical property of the saliva, and a computing part for evaluating the risk of possible caries based on the measured value for the chemical property of the stored saliva after a given ratio of acid is dropped in the saliva in the container.

The device of evaluating the risk of possible caries in the fourth mode of the present invention has such a characteristic that it is composed of a container for storing the saliva collected from the mouth of the subject, a sensor surface, which comes in contact with the saliva to measure the chemical property of the saliva, and a computing part for evaluating the risk of possible caries based on the difference between the measured value for the chemical property of the stored saliva immediately after the collection and that after a given ratio of acid is dropped in.

This means that the device of evaluating the risk of possible caries allows for more convenient evaluation of the risk of possible caries simply using a minute quantity of saliva. In addition, since the risk of possible caries is output in the form of grade, the precise picture of the result of evaluation can be easily understood.

If the computing part has a table for storing the relationship, which derives the grade of the risk of possible caries from the two measured values got before and after adding acid to the saliva or the measured value got after adding acid in the saliva, between the measured value(s) of chemical property of the saliva and the grade, more correct evaluation is achieved based on statistics derived by the measured value(s).

A system of evaluating the risk of possible caries in the fifth mode of the present invention has such a characteristic that it is composed of a measuring device for measuring the chemical property of the saliva collected from the mouth of the subject, a server having a database storing evaluation criterion data used in evaluating the risk of possible caries based on the measured value for the chemical property of the saliva to possess a risk evaluation function for evaluating the risk of possible caries for the subject using the measured value for the chemical property measured by the measuring device, a terminal having at least a evaluation output function for outputting the result of the evaluation of the risk of the possible caries, and a network for performing at least one of data inputs/outputs among the measuring device, the server, and the terminal.

For that reason, the dentist or patient can measure the chemical property of sample such as sallva collected from the mouth using the measuring device and obtain the grades of evaluation of the risk of possible caries. This means that the patient can accurately judge his/her own mouth condition based on the grade of evaluation of the risk of possible caries, and actively visit the dentist for treatment, if needed. In other words, the risk of possible caries, which has been difficult to determine conventionally, can be correctly recognized based on the measured values for the chemical property of the saliva and the need for treatment can be determined based on the grade of evaluation.

Note that the measuring device can be installed at, for example a dental clinic, though it may be located at the subject's home and connected to the server via a network. Similarly, the server may be installed at, for example a dental clinic, though it may be located at the administrating firm to administer the servers to connect a plurality of dental clinics to them via the terminals and the network. The terminal is installed at the dental clinic and/or the patient's home. In addition, the terminal needs not to be a so-called terminal for connecting to the network but may be an output device, for example a display (including a indicator of the measuring device), a printer, or an input device, for example a keyboard or a mouse which are directly connected to the server.

If the database contains electronic medical charts recording dental condition transition data composed of the measured value data for the chemical property of the saliva of the subject, it can be used in treatment because these measured values are accumulated in the database in the form of electronic medical charts. When in particular, the patient visits another dentist, he/she can provide the dentist with his/her electronic medical chart to establish the better communication with the dentist.

If the server has a electronic medical chart browsing function for browsing the dental condition transition data of the subject recorded on the medical chart by using the terminal, the subject can browse his/her own electronic medical chart to make efforts in keeping his/her mouth condition better. On the other hand, if the dentist browses the electronic medical chart of even new patient, he/she can recognize their mouth condition transitions and more correctly treat him/her. In addition, the dentist can give an appropriate advices to every patient on how to brush teeth, how to improve eating habit, and the recommended kind of tooth pastes to have resistivity to caries.

If the electronic medical chart contain the history of medical treatment of the subject teeth as dental condition transition data and the server has an evaluation criterion create/update function for creating or changing the evaluation criterion data by taking the statistic on the measured value data and the history data recorded on a plurality of electronic medical charts, the evaluation criterion data indicting the relationship between the measured values for the chemical properties of the saliva samples and the grades of evaluation of the risk of possible caries can be refined based on the statistics of the actual data to make more accurate evaluation of the risk of possible caries.

Note that if a researcher on the risk of possible caries participates in the study as an administrator of the data base or the statistics of the measured value data for the chemical properties of the saliva samples and the dental condition transition data are provided to the researcher, the study on this subject may be promoted. For the chemical properties relating to the risk of possible caries, the pH value or pH buffer capacity of the saliva samples may be considered at present, though other chemical properties may be measured in the future. This means that preferably, the kinds and values of the chemical properties to be used as the evaluation criteria are modified or added if needed based on advices by the researcher participating in the system for evaluating the risk of possible caries as an administrator or assistant.

If the system is composed of a terminal having an individual evaluation criterion input function for browsing the dental condition transition data recorded on the electronic medical chart to create or modify the individual evaluation criterion data refined for each subject, the evaluation criteria refined for the conditions of individual subjects can be created to more accurately determine the risk of possible caries.

If the database contains the treatment guideline data giving the guideline for treatment in accordance with the evaluation criteria, the dental disease can be treated in accordance with the grade of evaluation of the risk of possible caries, increasing the utility value of the system for evaluating the risk of possible caries.

If the server has a treatment recommendation distribution function for distributing appropriate treatment to the individual subjects in accordance with the grade of evaluation of the risk of possible caries via any of electronic communication means such as e-mail, the subjects can understand that their mouth conditions require medical treatment and undergo the treatment at an early stage without a loss of timing.

If the treatment guideline data contains the subject treatment guideline data for the subjects and the dentist treatment guideline for the dentists, the subjects can obtain the understandable treatment guideline written in plain words, while the dentists can obtain the definitive professional medical guidance, which help them improve their skill. In this context, the subject enjoys the safety when visiting the dentist as a patient because the dentist gives appropriate and easy explanations facilitating the communication between both of the dentist and subject sides.

If the database contains a mouth condition medicine list, the subjects can select and purchase the appropriate medicine for improving their mouth conditions with ease. In addition, pharmaceutical companies manufacturing the drugs can take the sales-promotion activities simply by registering the efficacy of their drugs in the mouth condition medicine list.

If the server has an inspection reservation function for accepting the evaluation of the risk of possible caries by assigning the right of use of the measuring device to the individual subjects when accessed by the subjects via their terminals via the network, the subjects can undergo the evaluation of the risk of possible caries at a convenient time by accessing the server via the network to reserve the measuring device, improving their convenience.

Note that the subject may measure his/her chemical properties using the measuring device at the subject's home by sending the measuring device to the subject from the administrator of the system for evaluating the risk of possible caries and the chemical properties of the saliva sample may be measured at the inspection laboratory or the administrator's site by sending the saliva sample collected from the mouth of the subject. Moreover, the subjects may visit the inspection site arranged by the dentist or the inspection laboratory having the measuring device to measure the chemical properties of their saliva samples.

If the server has an evaluation distribution function for distributing grades of evaluation of the risk of possible caries to the subjects via any of electronic communication means such as e-mail, the subjects can easily receive the grades of evaluation and treat appropriately according to them.

The server contains a dentist list, a dentist, who participates in the system for evaluating the risk of possible caries, may be introduced to the patient. For that reason, the subjects can easily identify the dentist, who correctly treats based on the correct determination using the system for evaluating the risk of possible caries.

If the server has a treatment reservation function for reserving the treatment by the dentist, who is included in the dentist list, by accessing from the subject's terminal via the network, the subject can reserve the treatment without the need for visiting or calling the dentist in the dentist list, improving his/her convenience. The dentists can accept their patients simply by registering in the system for evaluating the risk of possible caries of the present invention.

If the server has a regular inspection recommendation function for distributing the recommendation of regular inspection to the subjects depending on their grades of evaluation of the risk of possible caries via any of electronic communication means such as e-mail, it can be recommended that preferably, the subjects undergo the regular inspection at an interval adjusted for each of the subjects, which enables the subjects to eliminate the need for unwanted inspections and remember only the required ones.

The program of evaluating the risk of possible caries in the six mode of the present invention has such a characteristic that it involves a step for obtaining the measured value for the chemical property of the saliva collected form the mouth of the subject, a step for referring to the evaluation criterion data in evaluating the risk of possible caries based on the measured value for the chemical property contained in the database, and a step for outputting the obtained measured value for the chemical property in the form of the grade of evaluation of the risk of possible caries based on the evaluation criterion data, and that these steps are performed by a computer.

If using this program, the server obtains the measured values via the communication network or when an operator enters the sent measured data in the computer to obtain the measured values, refers to the evaluation criterion data contained in the database, makes the evaluation of the risk of possible caries, and outputs the grade of evaluation to the printer, display unit or communication network, the mouth conditions can be easily and accurately recognized. In this case, a function for storing the dental condition transition data of the subject containing the measured values for the chemical properties in the database as an electronic medical chart may be added to the program to be executed by the server.

The method of evaluating the risk of possible caries in the seventh mode of the present invention has such as characteristic that it measures the chemical property of the saliva collected from the mouth of the subject and outputs the measured value for chemical property in the form of the grade of evaluation of the risk of possible caries of the subject by referring to the database recording the evaluation criterion data for evaluating the risk of possible caries in accordance with the measured value, creates an electronic medical chart recording the dental condition transition data containing the measured value for the chemical property of the subject's saliva, and performs at least one of input of the measured value for the chemical property, reference to the database, and output of the grade of evaluation of the risk of possible caries through data communications via a network.

This means that by applying any information communications technology, convenience can be improved and the subject can accurately recognize their own mouth condition based on the measured values for the chemical properties,

### Brief Description of the Drawings

FIG. 1 is a view showing one example of a device of evaluating the risk of possible caries of the present invention.
FIG. 2 is a view explaining one example of a method of evaluating the risk of possible caries of the present invention.
FIG. 3 is a view showing the result of the measurement of the chemical properties of a plurality of saliva samples.
FIG. 4 is a view explaining another example of the method of evaluating the risk of possible caries.
FIG. 5 is a view showing the whole configuration of a system of evaluating the risk of possible caries of the present invention.
FIG. 6 is a view showing one example of a measuring device used in the system of evaluating the risk of possible caries.
FIG. 7 is a view showing one example of a screen, which can be browsed by subjects using a server of the system of evaluating the risk of possible caries.
FIG. 8 is a view showing another example of the screen.
FIG. 9 is a view showing further another example of the screen.
FIG. 10 is a view showing a screen, which can be browsed by dentists using the server of the system of evaluating the risk of possible caries.
FIG. 11 is a view explaining one example of a conventional method of evaluating the risk of possible caries.

### Best Mode for Carrying Out the invention

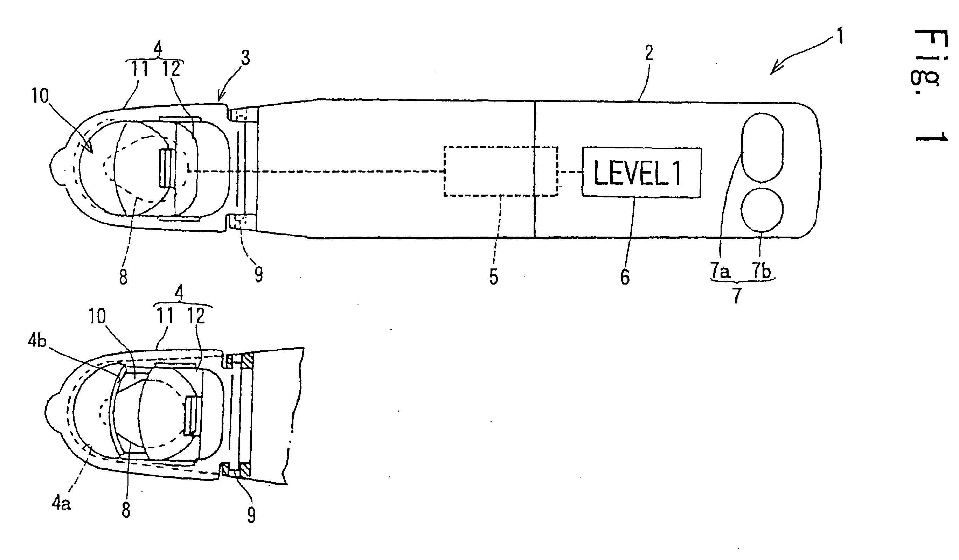

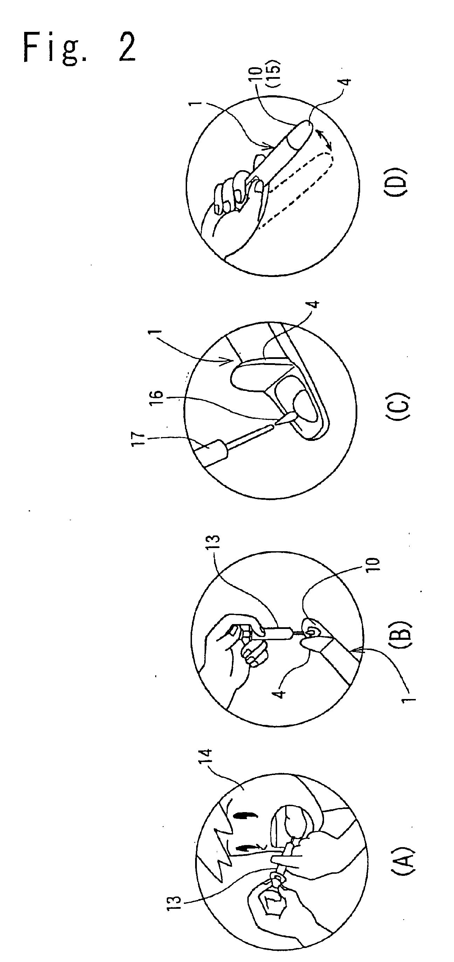

FIG. 1 is a view showing the whole configuration of one example of a device 1 of evaluating the risk of possible caries of the present invention. In FIG. 1, 2 indicates a main body, 3 indicates a sensor part, which can be detachably attached to the main body 2, and 4 indicates a rid, which can be detachably covered to the sensor part 3.

The main body 2 is composed of a computing part 5 for computing the grades of evaluation of the risk of possible caries using a pH value as one example of the chemical properties of saliva samples to be measured in the sensor part 3, a display part 6 for displaying at least the grades of evaluation of the risk of possible caries computed in the computing part 5, and a operating buttons 7 for operating the device 1 of evaluating the risk of possible caries. The operating buttons 7 include, for example a power button 7a and a measurement button 7b.

The sensor 3 is composed of a sensor surface 8, which has been made by arranging, for example an ISFET measuring electrodes and comparison electrode on the same plane and a container 10 for storing the saliva samples collected from the mouths of the subjects, which are constructed so that the sensor surface 8 forms its bottom. Note that the sensor surface 8 of the example is made from the ISFET measuring electrodes for pH measurement and the comparison electrodes because it shows the case where the grade of evaluation of the risk of possible caries is determined based on the pH measured values, though alternatively, instead of pH, ORP measured values or any of other chemical properties such as other ion (for example, calcium ion) concentration and protein quantity may be used as a determination criterion.

In addition, such a method may be considered that a plurality of chemical properties are measured to comprehensively determine the individual chemical properties for evaluating the risk of possible caries. It does without saying that electrodes composing the sensor surface 8 should be changed in the case where other solution property parameters including ORP and calcium ion are measured.

The use of ISFET in the sensor surface 8 enables the required quantity of the saliva sample 15 to be reduced, though the present invention does not limit the kind of ion electrodes composing the sensor 8 to ISFET and miniaturized regular glass electrodes or flat glass electrodes may be used.

The rid 4 is rotatably attached to the main body 2 by a hinge 9 and is composed of a rid main body 11 forming a sealing part 4a, which is watertightly engaged with the edge of the container 10 with the rid 4 closed and an opening 4b in the upper part of the container 10 and a movable element 12 for closely switching the opening 4b when slidably engaged with the rid main body 11. To prevent the saliva samples 15 from leaking from the container 10, it is an important factor to closely seal the container, though the present invention does not limit the configurations of the rid 4 and other members.

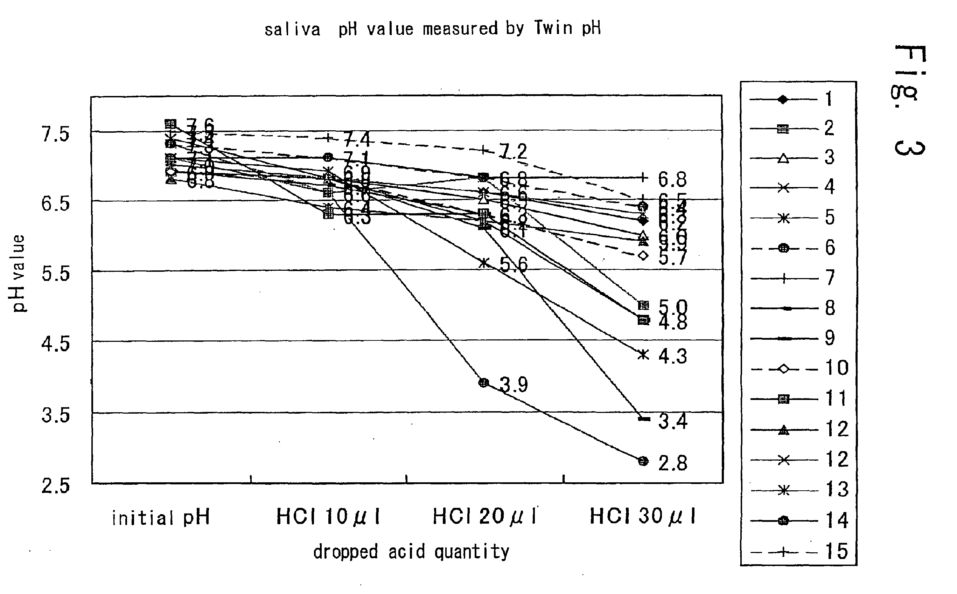

FIG. 2 is a view explaining the method of evaluating the risk of possible caries using the device 1 of evaluating the risk of possible caries. Now, one example of the method of evaluating the risk of possible properties is described below in reference to FIGS. 2 and 3. Namely, the use of the device 1 of evaluating the risk of possible caries enables the risk of possible caries may be evaluated easily. On the other hand, in the method of evaluating the risk of possible caries of the present invention does not limit to the use of the device 1 of evaluating the risk of possible caries.

First, as shown in FIG. 2A, for example a sterilized syringe 13 is used to collect the saliva sample from the subject 14. At that time, the quantity of the saliva sample to be collected is the quantity required for measurement, and it depends on the capacity of the sensor 3. If the ISFET sensor surface 8 is formed as shown in this example, it is satisfactory to collect 0.5 mL or more saliva and is checked at the divisions (not indicated) marked on the syringe 13. Note that the subject 14 may bite a paraffin palette 90 in the conventional manner to induce the secretion of saliva for collection.

As shown in FIG. 2B, the collected saliva 15 is checked at the divisions on the syringe 13 and 0.5 mL of saliva is dropped on the sensor surface 8, for collecting it in the container 10. At that time, the saliva sample 15 may be stored in the container 10 with the rid main body 11 open or collected it through the opening 4b by opening the removable element 12 with the rid main body 11 closed. Note that the present invention does not limit the quantity of the saliva sample 15 to be used in evaluating the risk of possible caries and more minute quantity of saliva sample 15 may be used for measurement depending on the performance of the senor surface 8.

Second, the operating button 7b is pressed to measure the pH value for the saliva sample 15. Note that the power on the device 1 of evaluating the risk of possible caries may be turned on prior to measurement or immediately before the pressing the operating button 7b. Alternatively, the initial pH measurement may be autonomously started when the saliva sample 15 begins to be dropped on the sensor surface 8.

As soon as pH measurement is finished, the pH value is stored and a message for requesting the drop of acid is output by issuing an acoustic or visual alarm to the operator.

FIG. 2C is a view showing a step for mixing a given ratio of acid 16 in the saliva sample 15. The acid 16 to be used here is 0.1 mol/L of hydrochloric acid solution and 10 µL is measured using a desired chemical feeder 17 for infusion. (Namely, 0,000002 g equivalent weight to the saliva sample 1 mL).

FIG. 2D is a view showing a step for mixing the infused acid 16 in the saliva sample 15. At that time, the container 10 can be hermetically closed by fully closing the rid main body 4 to prevent the saliva sample 15 from leaking when the device 1 of evaluating the risk of possible caries is well shaken in order to mix the saliva sample 15 and the acid 16 sufficiently.

It is desired that the time of the saliva sample 15 being left is set using the program to be executed in the computing part 5. This means that the computing part 5 waits for, for example 30 seconds since pressing the measurement button 7b and then outputs the message for requesting the second pH measurement by issuing the acoustic or visual alarm, allowing for stable measurement with no complex labor.

Then, as soon as the pH values has been measured immediately after the 10 µL of hydrochloric acid solution is mixed, the device 1 of evaluating the risk of possible caries outputs the message for requesting dropping of 10 µL of hydrochloric acid again to the operator to repeat the steps in FIGS. 2C and 2D. Thus, by dropping the hydrochloride acid three times and measuring any variation in pH value, any change in pH value is measured when 30 µL of hydrochloride acid solution (a given ratio of acid 16 by 0.000006 g equivalent weight to 1 mL of saliva) in total is mixed in 0.5 mL of saliva sample 15.

As shown in this example, by adding a 0.000002 g (equivalent weight) aliquot of acid 16 to 1 mL of saliva three times and measuring any variation in pH value each time, the pH buffer capacity of the saliva sample 15 can be more accurately determined. However, it goes without saying that a given ratio of acid 16 may be dropped at the same time for obtaining the final result. In this case, the time required for measurement is shorten.

Finally, the saliva sample 15, which has been used for pH measurement after the mixture of acid 16, is discarded and the inside of the container 10 is washed to prepare for the measurement of the next saliva sample. Namely, this method requires no disposable inspection elements, for example a strip 92 in the conventional method, reducing the cost of measurement.

FIG. 3 is a view showing the result of the measurement of any variation in pH when 0.1 mol/L hydrochloride acid solution is dropped by 10 µL for mixing in the 0.5 mL of saliva sample 15 collected from each of 15 subjects. As shown in FIG. 3. when 30 µL of hydrochloride acid solution was dropped, a large variation was observed in pH value of the saliva sample 15 for each subject. which may be measured to use a marker of the buffer capacity of each of the saliva samples 15. Namely, based on the variation in pH value of the saliva sample 15, the pH buffer capacity of the saliva sample 15 for each subject can be accurately evaluate.

The evaluation may be performed by referencing to a table recorded in the computing part 5 or a recording medium in other part. The table given in this example describes the staged grades of evaluation of the risk of possible caries for the pH values of the saliva samples 15 measured after acid was dropped and specifically, it can be determined that the saliva sample 15 with 5.7 or higher pH, when measured after 30 µL of hydrochloride acid solution being dropped, has a statistically lower risk (LEVEL0) of possible caries. It also can be determined that the sallva sample 15 with 5.4 or higher and 5.7 or lower pH has a medium risk (LEVEL1) of possible caries and the saliva sample 15 with pH lower than 5.3 has a higher risk (LEVEL2) of possible caries. Note that these ranges described here do not limit not only the scope of the present invention but also the risk of possible caries to the three-level representation.

In any cases, since the pH value measured after 30 µL of hydrochloride acid solution being dropped indicates the buffer capacity of the saliva sample 15 to acid, the risk of possible caries may be easily determined based only on the pH value measured after 30 µL of hydrochloride acid solution being dropped. However, the present invention does not limit the evaluation of the risk of possible caries to the use of the pH value measured after 30 µL of hydrochloride acid solution being dropped.

This means that the table does not limit the computation of the staged grades of evaluation of the risk of possible caries to the use of pH value measured after acid was dropped. Namely, the risk of possible caries may be evaluated taking the pH value measured before acid was dropped into account together with that measured after acid being dropped. In other words, based on the pH value measured before acid was dropped, the risk of possible caries may be evaluated in the usual state and the properties of saliva secreted by the subject may be evaluated using the paraffin pellet 90 and the like. Based on any difference between the pH values measured before and after acid was dropped, the risk of possible caries can be evaluated more accurately. In addition, various modes of tables may be considered, for example to enable detailed evaluation based on the time-course change in pH after acid was added.

Furthermore, the grades of evaluation of the risk of possible caries are not limited to one-digit representation. Namely, the incident factor of caries in the usual state may be derived based on the initial pH value to derive the incident factor of the resistivity to caries based on the buffer capacity to acid 16. In any cases, by rapresenting the risk of possible caries in the form of the grade of evaluation of the risk of possible caries rather than in the form of pH measured values, the operator can determine easily the risk of possible caries.

Besides, the present invention does not the quantity of acid 16 to be dropped on the saliva sample 15 to its optimal condition (0.000006 g equivalent weight to 1 mL of saliva). It is understood from FIG. 3 that since even though the quantity of acid 16 to be mixed in the saliva sample 15 is reduced to lower than the value mentioned above, for example 20µL of 0.1 mol/L hydrochloride acid solution is dropped on 0.5 mL of saliva sample 15 (0.000004 g equivalent weight to 1 mL of saliva), the pH value of the saliva sample 15 with higher risk of possible caries decreases due to the influence of mixed acid 16, this ratio of acid 16 is uniformly dropped on all the saliva samples 15 to evaluate the risk of possible caries.

Even if the quantity of acid 16 to be mixed in the saliva sample 15 is increased compared with that mentioned about (0.000006 g equivalent weight to 1 mL of saliva), the risk of possible caries may be evaluated in the range where the saliva sample with lower risk of possible caries exhibits the pH buffer capacity to mixed acid 16 by dropping the same given ratio of acid 16 on all the saliva samples 15.

Again, although the example describes the use of the hydrochloride solution as an example of acid 16, the present invention does not limit the kind of acid 16 only to hydrochloride acid. In the case of hydrochloride acid, 0.1 mol of hydrochloride acid is mixed in the 1 L of solvent, though it goes without saying that the quantity of other acid 16 depends on its property to chemical reaction.

In the example mentioned above, the method of evaluating the risk of possible caries using the device 1 of evaluating the risk of possible caries having the function for evaluating the risk of possible caries has been described, though in the method of evaluating the risk of possible cries, any of standard devices of measuring the chemical properties may be used such as a commonly used pH measuring device. In this case, a correspondence between pH measured values is provided as shown in FIG. 3 for the operator to refer to it to evaluate the risk of possible caries based on the two pH measured values using the commonly used pH measuring device converted into the grades of evaluation.

By forming a storing part for acid 16 and a dispensing part for acid 16 on the device of evaluating the risk of possible caries as shown in the example mentioned above, a given quantity of acid 16 may be automatically dispensed on the saliva samples 15. In this case, the operator needs not use extra devices such as a drug solution injector 13 for acid addition. Furthermore, by attaching a sensor for measuring the quantity of the saliva sample 15, the quantity of acid 16 may be automatically regulated to that of the saliva sample 15.

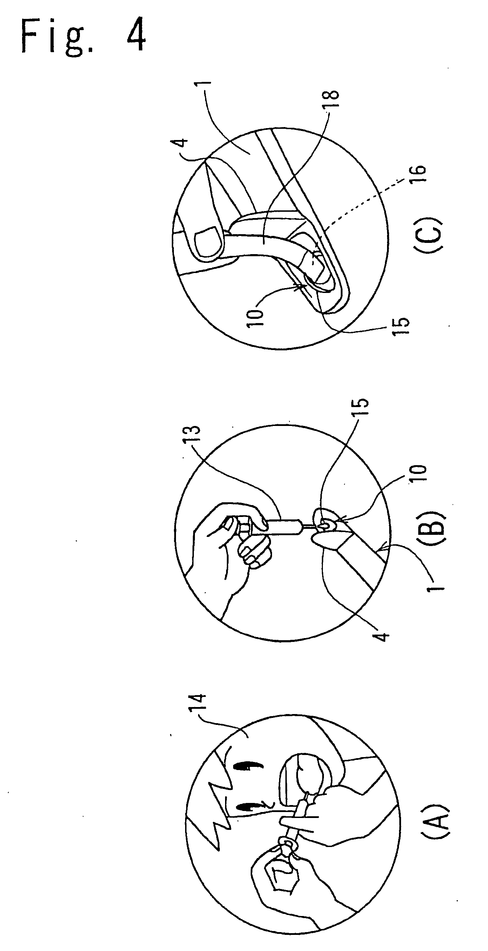

FIG. 4 is a view showing an example of another technique for mixing acid 16 in the method of evaluating of possible caries.- FIGS. 4A and 4B are the same as FIGS.2A and 2B, each showing the step for collecting the saliva samples 15 and the step for dropping the collected saliva samples 15 on the device 1 of evaluating the risk of possible caries.

FIG. 4C shows a step for mixing acid 16 in the saliva samples 15 in the container 10 using the absorbing pieces soaked with acid 16. 18 indicates a test strip as an example of the absorbing piece and the test strip 18 has been soaked with 0.000003 g equivalent weight of acid 16 so that a given ratio to 0.5 mL of saliva may be established. For that reason, simply by soaking the test strip 18 in the saliva samples 15, an accurate given ratio of acid 16 may be mixed in the saliva samples 15.

Then, the pH values of the saliva samples 15 with acid 16 mixed is measured to evaluate the risk of possible caries. As shown in this example, the use of the test strip 18 makes the mixture of the given ratio of acid 16 in the saliva samples 15 easier, further saving the operator's labor.

Note that in the examples mentioned above, the techniques for dropping the aqueous acid solution have been described, though the present invention does not limit acid only to this type of phase. Namely, such techniques may be used that a given quantity of acid in the solid phase at room temperatures such as citric acid is added after transformed into powder or pellets.

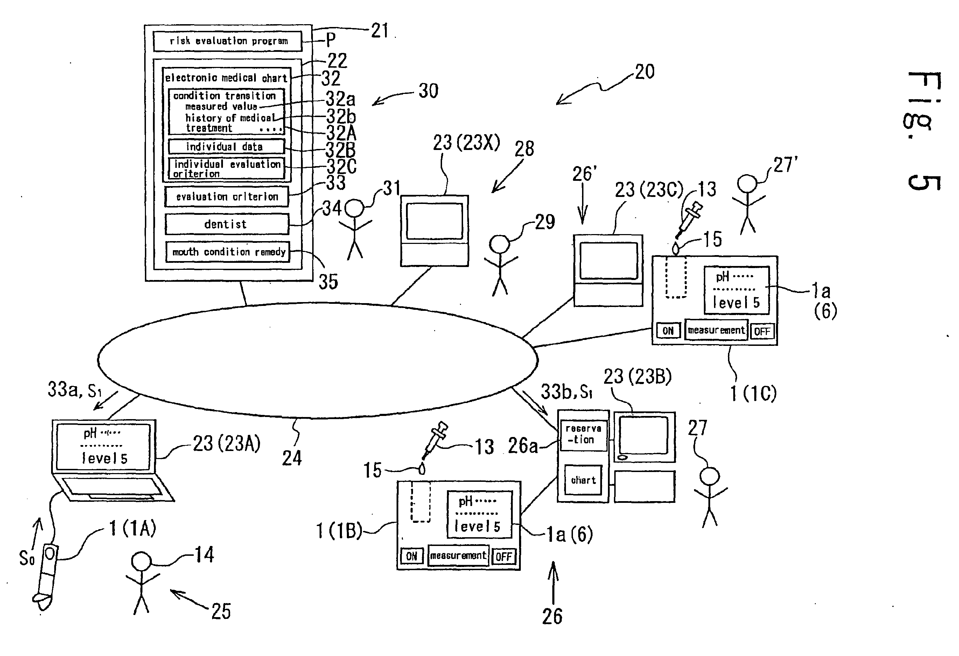

FIG.5 is a view showing one example of a system 20 of evaluating the risk of possible caries of the present invention. The descriptions of the portions in FIG. 5 having the same symbols as those in FIG. 4 are omitted because they are the same or equivalent portions. 1A indicates a handy (stick) type of measuring device for the subject 14 to measure the chemical properties of the saliva sample collected from his/her own mouth.

In FIG. 5, 21 indicates a server for evaluating the risk of possible caries for the subject 14 from measured chemical properties, 22 indicates, for example a database concluded in memory of the server 21, 23 indicates a terminal having a function for outputting a evaluation of the risk of possible caries, and 24 indicates the internet as one example of networks connecting among the measuring device 1A, 1B, 1C··· the server 21, and the terminal 23.

The terminal 23 in this example having, for example any of information processing devices such as a PC and server, is composed of the terminal 23A installed at the home 25 of the subject 14, the terminal 23B installed at a dental cilnic 26 for the dentist 27 to operate, the terminal 23C installed at the inspection firm 26' for the laboratory technician 10' to operate for measuring chemical properties of the samples collected from the mouths of the subjects 14, and the terminal 23X installed in the laboratory for a researcher 28, who makes a study on dental sanitation, for scholars 29 to operate and each terminal 23A, 23B, 23C and 23X may be installed in various places connected via the internet 24.

On the other hand. the server 21 in this example is for executing a program P of evaluating the risk of possible caries and is an information processing device installed at a database administration firm 30 for making inspections on the risk of possible caries and administrating the database 22, and it is operated by an administrator 31 of the database 22. However, the servers 21 may be installed at each of the dental clinic 26 or the inspection firm 26' and administrated by the dentist 27 or the laboratory technician 10'. In this case, the existing information processing devices installed in the dental clinic 26 and the inspection firm 26' (in this example, terminals 23B and 23C), if any, may be used as the servers 21.

It goes without saying that the database 22 is not necessarily recorded in memory of the server 21 and another server may be added for administrating the database 22. On the other hand, as shown in this example, if the administration firm 30 administers the database 22, information can be managed beyond the extent of the dental clinic 26 and a wide variety of information useful in evaluating the risk of possible caries can be collected as described later.

The measuring devices 1A, 1B and 1C measure the pH values and pH buffer capacities of the saliva samples collected from the mouths of the subjects 14 as chemical properties. Various types of measuring devices 1A, 1B and 1C may be used, and a stick type of measuring device 1A, which has been designed so that the subject 14 can connect them to his/her terminal 23A via a given standard of interface, for example USB at his/her home, can be given an example.

For the types of the measuring devices 1A, 1B and 1C any stationary measuring device 1B can be used, which has been designed so that it may be connected to the terminals 23B and 23C via the given standard of interface, for example USB, installed at the dental clinic 26. As the measuring devices 1B and 1C shown in FIG. 5, a display 1a having the function for displaying the measured values for the chemical properties or outputting the grades of evaluation of the risk of possible caries can be used to serve also as the terminal 23.

In addition, the measuring device 1B may have the same function as that of the terminal 23 by connecting it directly to the internet 24. Furthermore, the measuring device 1C administered by the inspection firm 26' may be used instead.

The database 22 is composed of electronic medical chart 32 containing the month condition transition data 32A, individual data 32B on the subject 14, and the individual evaluation criterion data 32C described later, the evaluation criterion data 33 indicating at least the relationship between the measured values for the chemical properties and the grades of evaluation of the risk of possible caries, the registered dentist data 34 indicating the information on the registered dentists cooperating in the system of evaluating the risk of possible caries of the present invention, and the mouth condition medicine data 35 indicating the information on the recommended mouth condition remedies.

The mouth condition transition data 32A is composed of the measured value data 32a consisting of, for example the history of the measured values for the chemical properties of the saliva samples collected form the mouths of the subjects 14 and the history of dental treatment data 32b of the subjects 14, which are medical treatment data specific to the subject 14, where the dentist 27 adds the descriptions. The individual data 32B is composed of, for example the information on the addresses, names, ages, genders and contacts (including e-mail addresses) of the subjects 14. In addition, the individual evaluation criterion data 32C is a group of individual evaluation criteria refined for each of the conditions of the subjects 14.

For the evaluation criterion data 33, not only the data indicating the relationship between the measured values for the chemical properties and the grades of evaluation of the risk of possible caries, but also, for example the statements "urgent treatment is needed", "the diagnosis by a dentist is recommended", "the regular inspection is recommended", and "no risk of possible caries is observed at present" and the guideline for preventing caries from developing, for example the number of tooth brushing a day may be included. It is desirable that the general treatment guideline 33a, which is easy for the subjects 14 to recognize and the professional treatment guideline 33b, which provides the dentist with a plenty of information described later are individually included in treatment guideline.

Moreover, similarly, it is desired that the treatment guideline data in the form of statements is contained in the evaluation criteria recorded as the individual evaluation criterion data 32C, and the general treatment guideline, which is easy for the subjects 14 to understand, and the professional treatment guideline, which provides the dentists with a plenty of information are individually included.

Note that in any cases, in this example, the treatment guideline data has been included for outputting the treatment guideline (statements) corresponding to the grades of evaluation as part of the evaluation criterion data, though they may be separated into two parts and stored in the database.

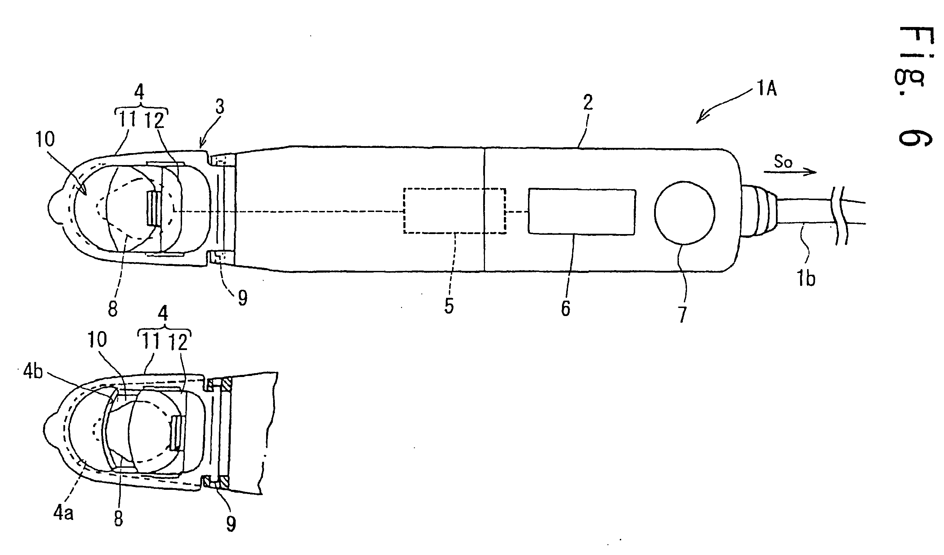

FIG. 6 is a view explaining one example of stick type of measuring devices 1A. Note that since the measuring device 1A in this example composes part of the device 1 of evaluating the risk of possible caries shown in FIG. 1, the duplicated explanation of the same or equivalent portions are avoided using the same symbols as those in FIG. 1.

In FIG. 6, 1b indicates a cable connecting a main body 2 of the measuring device 1A to the terminal 23A (refer to FIG. 5) via for example USB. Since the measuring device 1A in this example is power-supplied while connecting to the terminal 23 A, only a measurement button is satisfactory for the operating button 7.

The measured values S₀ for the chemical properties measured by the sensor surface 8 of the measuring device 1A are designed so that they may be sent to the terminal 23A via the signal cable 1b and sent to the server 21 via the terminal 23A.

In the case where the subject 14 shown in FIG. 5 accesses the server 21 via their own terminals 23A to submit the application for using the system of evaluating the risk of possible caries, they can create the medical charts 32 of the subject 14 in the database 22 and receive the measuring device 1 from the inspection firm 26' (or from the dentist 10 or the database administration firm 30). In this case, to create the correct electronic medical chart 32, the history of dental treatment of the subject 14 and the like should be browsed on the web or the written medical records sent. At the same time, the 1D or password of the subject 14 is determined, which are needed in using the system 20 of evaluating the risk of possible caries of the present invention.

Namely, the server 21 has an inspection reservation function for accepting the grades of evaluation of possible caries and assigning the right of use of the evaluation system server 21 of the present invention to each of the subjects 14 when accepting the application for using the system and sending the usable measuring device 1A to the subject 14. Note that the application for using the evaluation system is not limited to the use of the internet 24 and may be made by sending a given form of application, in which required data is filled by the subject 14, to the inspection firm 26'.

The subject 14 connects the measuring device 1A, once received at hid/her home 25, to the terminal 23A and measures the chemical properties of the saliva samples collected from his/her own mouth. Since the method of measuring the chemical properties using the measuring device 1 has been described in reference to FIGS.2 and 4, the detailed explanations of it is omitted here.

The subject 14 presses the operating button 7 to measure the pH value of his/her saliva sample 36. At that time, as shown in this example, since power is supplied to the measuring device 1A from the side of terminal 23A, the operation of the power source can be eliminated or streamlined, though it goes without saying that another power source may be added.

Once the pH value has been measured, the computing part 5 stores the measured pH value and then the message requesting the subject 14 to drop acid 16 supplied with the measuring device 1A from the inspection firm 26' on the saliva sample 15 with a acoustic or visual alarm. Then, the subject 14 drops acid 16 on the saliva sample 15, well shakes the measuring device 1 for mixture, and leaves it for a given time (approx. thirty sec. to one min.) to make the second measurement of pH value,

It is desirable that the time for which the sample is left is set using the program to be executed in the computing part 5. Thus, by dropping hydrochloride acid solution on the saliva sample three times and measuring any variation in pH value each time, a change in pH value is measured immediately after 30 µL of hydrochloride acid solution (a given ratio of acid 16 by 0.000006 g equivalent weight to 1 mL of saliva) has been mixed in 0.5 mL of saliva sample 15 to measure pH value and pH buffer capacity of the saliva sample 15.

The measured values S₀ such as the measured pH value and pH buffer capacity are accumulated in the database 22 in the server 21 via the terminal 23A and the internet 24, and the server 21 outputs the measured values S₀ for chemical properties to the terminal 23A after converting them into the grades S₁ of evaluation of the risk of possible caries and the treatment guideline 33a in reference to the evaluation criterion data 33 using the program P of evaluating the risk of possible caries.

The evaluation criterion data 33 indicates a relationship obtained based on the actual measured values as detailed above with reference to FIG. 3, which has been represented in the form of a table.

Note that an individual difference may be observed in the development of the pH buffer capacity after acid 16 has been dropped. Thus, using the system 20 of evaluating the risk of possible caries of the present invention, the condition transition data 32A of a plurality of subjects 14 is used to compare between the measured values for chemical properties of the collected saliva samples and the history of dental treatment of each of the subjects 14, which allows for creating of or updating to more actual evaluation criterion data 33. On the other hand, based on the history of treatment for each of the subjects, the individual evaluation criterion data 32C may be created taking their unique characteristics into account.

The individual evaluation criterion data 32C may be created or updated by the dentist 27, who examined the subject 14, entering data from the terminal 23B, to store it in the database 22. Then the accurate individual evaluation criterion data 32C is created by a high ability of the dentist 27. At the same time, additional data such as the treatment guideline represented by means of statements may be also included by the dentist 27.

Note that as shown in FIG. 5, the grads S₁ of evaluation of the risk of possible caries may be distributed to the subject 14 in the form of e-mail. Namely, the server 21 has the function for distributing the grades of evaluation of the risk of possible caries to the subject 14 using e-mail and the like.

In addition, the server 21 preferably has the function for distributing the recommended treatment to send the treatment guideline 33a containing an appropriate advice and the recommended treatment to the subject 14 with a lower grade S₁ of evaluation of possible caries by e-mail and the like.

Note that the measuring device 1 of the present invention is not limited to those shown in the examples mentioned above and those may be used which has a sensor for measuring the chemical properties and actually obtains the measured values. For that reason, the measuring device 1A of which design is simplified and disposable, may be used. In this case, the measuring device 1A enjoys an advantage that it can make the best measurements at a high accuracy without need for maintenance. On the contrary, like the measuring devices 1B and 1C shown in FIG. 5, a stationary type of measuring device, which is robust, may save the cost of singie measurements.

The subject 14 can access the server 21 using the terminal 23A and enter his/her 1D or password to browse his/her electronic medical chart 32. This means that the sever 21 has the function for browsing the electronic medical charts using the terminal 23A to browse the mouth condition transition data of the subject 14 consisting of the measured values 32a for the chemical properties and the history of treatment 32b and the like.

It is desirable that the subject 14 having the 1D and password can access the server 21 to accumulate the questions about how to keep the mouth condition better, which are to be consulted with the dentist, in the database 22 and the administrator 31 can distribute the appropriate answers to the questions accumulated in the database 22 via e-mail. Namely, it is preferable that the server 21 has the mouth condition consultation function for not only accumulating the questions about how to keep the mouth condition better but also distributing the advices by the professionals to the subject via e-mail and the like.

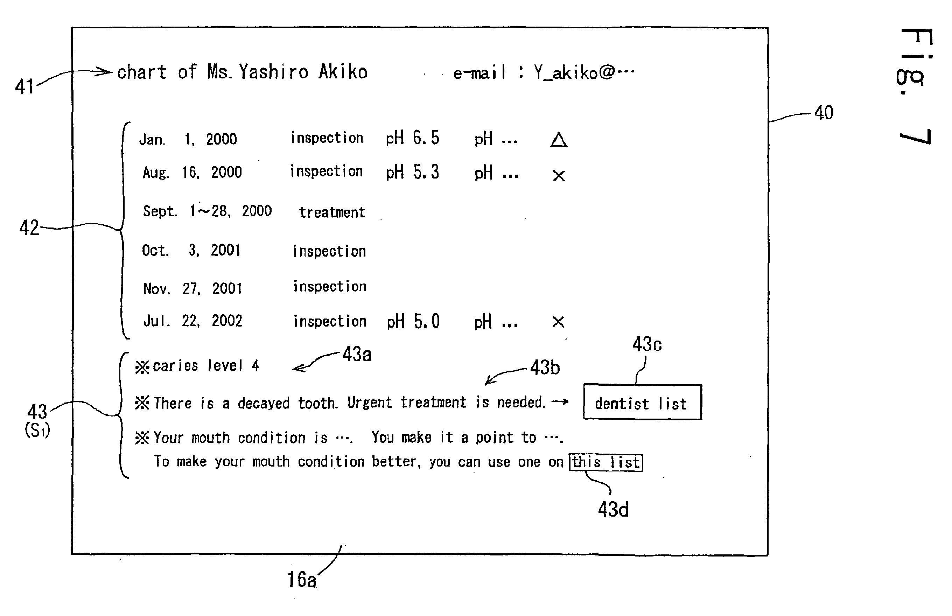

FIG. 7 is a view showing the example of the screen 40 displaying the electronic medical chart 32, which can be browses by the subject 14 when accessing the server 21. In FIG. 7, 41 indicates the individual information 328 such as the name and contact such as an e-mail address of the subject 14, 42 indicates the history of treatment such as the results of inspections, which have been made by the subject 14 so far and the mouth condition transition data 32A such as the measured values for the chemical properties obtained at the points when the inspections were made, and 43 indicates the grades of evaluation of the risk of possible caries for the subject 14.

The history of treatment 42 is used for disclosing briefly both of the measured value data 32a and the history of treatment data 32b to the subject 14 including the dates of inspections or treatments and their descriptions of them. The subject checks the screen, on which the history of treatment 42 is displayed, to recognize the transition in his/her mouth condition.

The grades of risk evaluation 43 includes the grades of evaluation of the risk of possible caries 43a and the treatment guideline 43b, which are derived based on the measured values S₀ for the chemical properties obtained from the last inspection made by the server 21 taking the individual information on the subject 14 into account. In addition, since the treatment is recommended on the treatment guidance 43b in this example, a link button 43c to the dentist list display screen and the link button 43d to the mouth condition medicine list display screen appear on the screen. It is well understood that the subject 14 can clearly understand his/her own-mouth condition and required treatment simply by browsing the risk evaluation 43.

The grades 43a of evaluation of the risk of possible caries may be not only based on the evaluation criterion data 33 but also based on the individual evaluation criterion data 32C described later if it has been input.

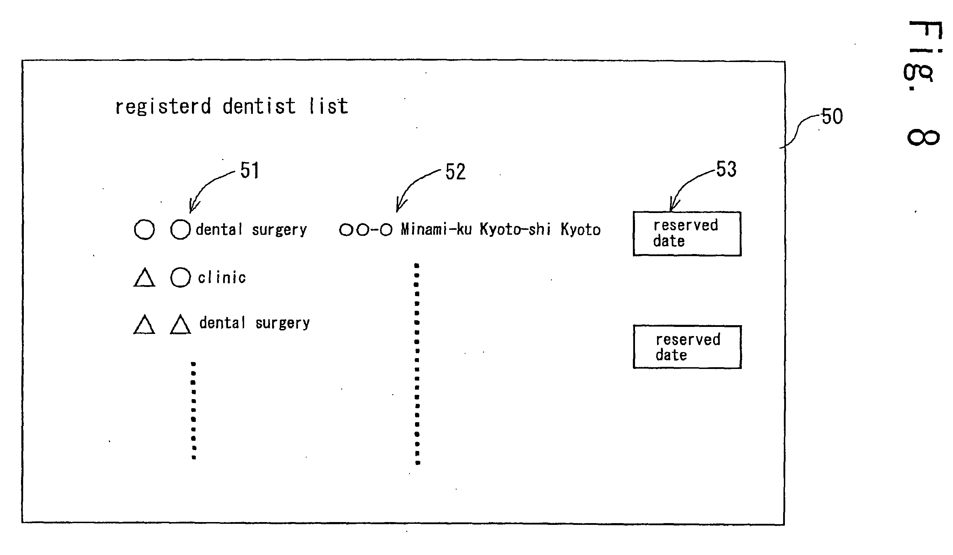

FIG. 8 is the screen 50 displaying the registered dentist list appearing when the subject 14 presses the link button 43c. In FIG. 8, 51 indicates the screen displaying the registered dental clinic name list recorded in the database 22 as the registered dentist data 34, 52 indicates the address list of dental clinics, and 53 indicates the reserved date reference button for each dental clinic. Note that these screens displaying these lists 51 and 52 are preferably arranged starting from the closest one to the address of the subject 14.

This means that the subject 14 can select the dental clinic close to his/her address, which treats correctly him/her using the system 20 of evaluating the risk of possible caries simply by browsing the screen displaying the registered dentist list 50. On the other hand, the dental clinics may accept the patients by registering in the database 22.

By creating the reservation date reference button 53 on the screen 50 displaying the registered dentist list, the subject 14 can check the reservation status of the selected dental clinic. In this case, the server 21 receives the reservation date information 26a (refer to FIG. 5) recorded in the terminal 23B installed at the dental clinic 26 via the internet 24, discloses it to the subject 14 and transfers the reservation contents on the subject 14 to the terminals 23B, 23C ··· isntalled at the dental clinics 26 to mediate between the patients and the dentists in the dentist list. Namely, the server 21 has the treatment reservation function which can accepts the application for treatment by a dentist by mediating between the patients and the dentists in the registered dentist data 17 when the subject 14 accesses from the terminal 23A via the network.

Thus, the convenience of the subject 14 increases because the subject 14 may reserve the desired date for treatment without the needs for visit to or calling up the dental clinic.

For that reason, on the dentist 27 side, the subject 14, who has made a query, may be easily identified using the system of evaluating the risk of possible caries and the subject 14 can undergo the appropriate treatment without additional labor because the dentist may refer to the history of treatment recorded in the electronic medical chart.

Note that in the case where the dental clinic 26 has established its own home page to allow the patients to reserve the treatment via the internet 24, using a link to the home page of the dental clinic 26, the mediation can be performed between the patents and the dental clinics using the treatment reservation function.

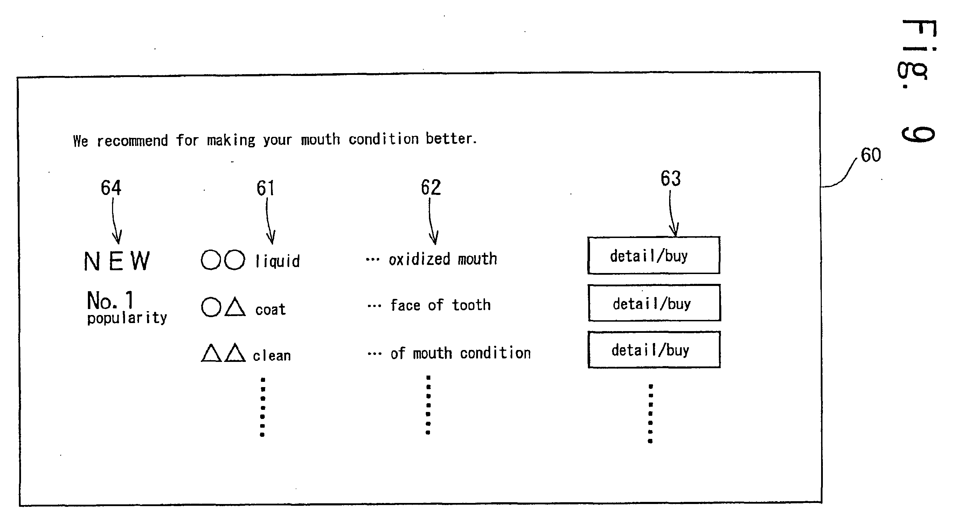

FIG. 9 is a view showing the screen 60 displaying the mouth condition medicine list appearing when the subject 14 presses the link button 43d from the screen 40 displaying the electronic medical chart 32 shown in FIG. 7. In FIG. 9, 61 indicates the list of the names of the mouth condition remedies recorded in the database 22 as mouth condition medicine data 35, 62 indicates the list of efficacies of the mouth condition remedies, and 63 is a button for displaying the detailed information on the mouth condition remedies or for purchasing them. Note that the server 21 preferably displays the contents of the mouth condition medicine list starting from the one with the highest efficacy.

This means that the subject 14 can very easily identify and purchase the necessary medicine for improving his/her mouth condition. On the other hand, the pharmaceutical company can accept queries about their drugs by registering the efficacies of the developed drugs in the database 22 as the mouth condition medicine data 35. Note that those referred to as "drugs" in the present invention include various types of mouth care products used in treating and preventing caries. Moreover, as indicated by a symbol 64 in FIG. 9, the comments by the administrator 31 of the database 22 may be annotated to the data.

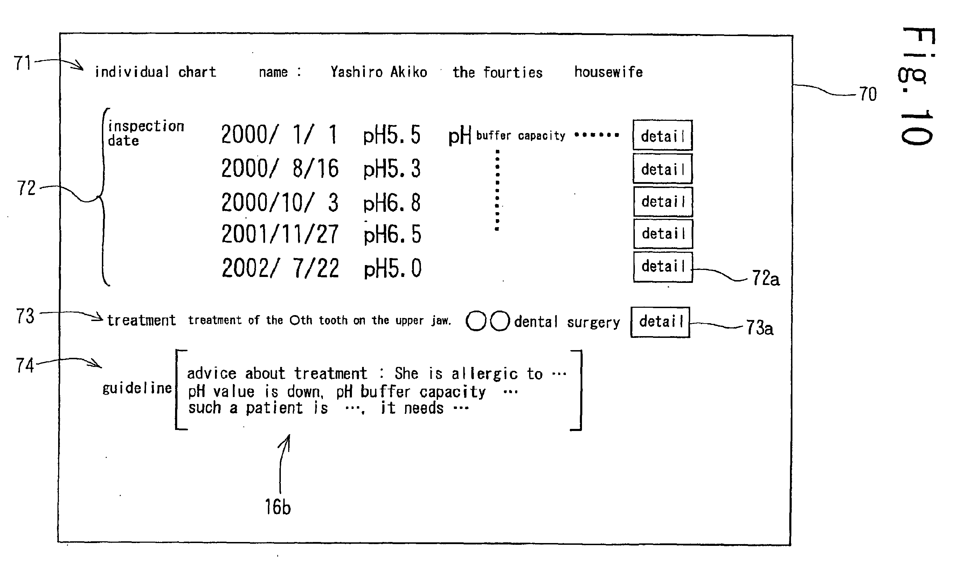

FIG. 10 is a view showing the screen 70 displaying the electronic medical chart for the subject 14 obtained when the dentist 27 accesses the sever 21 via the terminal 23B. In FIG. 10, 71 indicates the minimum individual data 32B of the subject 14 including his/her name, age, and occupation which are necessary for treatment, 72 indicates the history of inspections which the subject 14 have undergone so far, 73 indicates the history of treatment which the subject 14 have received so far, and 74 indicates the treatment guideline for the subject 14.

The screen 70 displaying the electronic medical chart in this example is different from the screen 40 displaying the electronic medical chart shown in FIG. 7 in that the former has not only a button 72a for displaying the detailed information on the inspections and a button 73a for displaying the detailed information on the treatments but also contents of the treatment guideline 74 is satisfactory for the dentist 27. For that reason, the dentist 27 can obtain accurate information on the subject 14, even though he visits him for the first time, to treat the subject 14 correctly,

Furthermore, the subject 14, who visits the dental clinic 26 as a patient, can undergo the appropriate treatment and establish the smooth communication with the dentist 27 without the need for providing the dentist 27 with the detailed information on himself/herself. In addition, in the case where the subject 14 visits another dentist due to the change his/her residence to another one, the subject 14 can undergo the treatment by the new dentist with safety because he/she can have a sufficient of knowledge of the subject 14.

The dentist 27 can enter the detailed information (including the treatment date and the therapeutic method used) on the result of treatment using the terminal 23B after the treatment to update his/her information in the electronic medical chart. Moreover, the dentist 27 can modify the evaluation criterion for the subject 14 if necessary and register it as the individual evaluation criterion data 32C in the electronic medical chart. In addition, the treatment guideline data can be as additional data to this individual evaluation criterion data 32C for giving an appropriate advice to the subject 14.

Note that as known from this example, it is preferable that for the dentist 27 to obtain the detailed information on the inspections, treatments, and treatment guideline, both of the information to be disclosed to the subject 14 and the information to be disclosed to the dentist 27 are accumulated. For the treatment guideline data 33, in particular, both of the subject treatment guideline data, which is written in the language easily understood by the subject 14, and the dentist treatment guideline, which gives the dentists with definitive information, are recorded.

Besides, it is also preferable that the treatment guideline 33 is created and updated so that it can be made more and more practical the contents of the database 22 improves. This means that if the detailed information on the evaluation criteria is gotten by taking statistic of the measured. value data 32a for the chemical properties and the history of dental treatment data 32b, then multi-levels of risk evaluation can be performed, the treatment guideline data corresponding to the results of the multi-levels of risk evaluation can be added.

In addition, by taking statistic of the individual evaluation criterion data 32C to which the dentist 27 added information and the condition transition data 32A, the database administrator 31 and the researcher 29 can create or update the evaluation criterion data 33, which is made more practical.

Moreover, if it is determined to be desirable to measure the chemical properties other than pH in the future, the new chemical properties can be added by replacing the sensor part 21 shown in FIG. 6.

Furthermore, the interval, at which the subject 14 undergoes the treatment, can be adjusted if necessary. Namely, if the server 21 has the regular inspection recommendation function for distributing the regular inspection recommendation to the subject 14 via e-mail and the like, the subject 14 can undergo the appropriate treatment before the caries become severe simply by receiving the inspection at an adequate interval.

Note that the e-mail messages are preferably distributed on the day as close as the day on which the inspection will be made. Thus, the subject 14 can undergo the regular inspection on a schedule.

### Industrial Applicability

As mentioned above, according to the method of evaluating the risk of possible caries of the present invention and the device of the same, the risk of possible caries can be easily and promptly evaluated, saving the operator's (or the user's) labor and time. In addition, since the chemical properties of saliva can be measured using electrical techniques, enabling repetitive measurement, which saves the running cost. In addition, the method of evaluating the risk of possible caries of the present invention is sanitary because the laboratory technicians need not contact directly the saliva samples.

Furthermore, according to the system of evaluating the risk of possible caries of the present invention, and the method, and the program of the same, the subject can conveniently undergo the evaluation of the risk of possible caries to obtain the appropriate treatment guideline. In addition, smooth communication may be established between the new subject and the dentist, being useful for both of the patients and dentists.

## Claims

1. A method of evaluating the risk of caries having such a characteristic that saliva is collected from the mouth of subject, a given ratio of acid is added to the saliva, and the chemical property of the saliva is measured to evaluate the risk of possible caries base on the measured value.

2. A method of evaluating the risk of caries having such a characteristic that the saliva is collected from the mouth of the subject, the chemical property of the saliva is measured using a chemical property measuring device, a given ratio of acid is added to the saliva, and the chemical property of saliva is measured again to evaluate the risk of possible caries based on the difference between the measured value of chemical property of saliva before adding acid and that after adding acid.

3. The method of evaluating the risk of caries according to claim 1 or 2, further comprising that acid is mixed in the saliva by determining the quantity of the saliva to be collected and immersing an absorbing piece, which absorbs a given ratio of acid, in the saliva.

4. A device of evaluating the risk of caries having such a characteristic that it is composed of a container for storing the saliva collected from the mouth of the subject, a sensor surface, which comes in contact with the saliva to measure the chemical property of the saliva, and a computing part for evaluating the risk of possible caries based on the measured value for the chemical property of the stored saliva after a given ratio of acid is dropped in the saliva in the container.

5. A device of evaluating the risk of caries having such a characteristic that it is composed of container for storing the saliva collected from the mouth of the subject, a sensor surface, which comes in contact with the saliva to measure the chemical property of the saliva, and a computing part for evaluating the risk of possible caries based on the difference between the measured value for the chemical property of the stored saliva immediately after the collection and that after a given ratio of acid is dropped in.

6. The device of evaluating the risk of caries according to claim 4 or 5, further comprising that the computing part has a table for storing the relationship, which derives the grade of the risk of possible caries from the two measured values got before and after adding acid to the saliva or the measured value got after adding acid in the saliva, between the measured value(s) of chemical property of the saliva and the grade.

7. A system of evaluating the risk of caries having such a characteristic that it is composed of a measuring device for measuring the chemical property of the saliva collected from the mouth of the subject, a server having a database storing evaluation criterion data used in evaluating the risk of possible caries based on the measured value for the chemical property of the saliva to possess a risk evaluation function for evaluating the risk of possible caries for the subject using the measured value for the chemical property measured by the measuring device, a terminal having at least a evaluation output function for outputting the result of the evaluation of the risk of the possible caries, and a network for performing at least one of data inputs/outputs among the measuring device, the server, and the terminal.

8. The system of evaluating the risk of caries according to claim 7, further comprising that the database contains electronic medical chart recording dental condition transition data composed of the measured value for the chemical property of the saliva of the subject.

9. The system of evaluating the risk of caries according to claim 8, further comprising that the server has a electronic medical chart browsing function for browsing the dental condition transition data of the subject recorded on the medical chart by using the terminal.

10. The system of evaluating the risk of caries according to claim 8 or 9, further comprising that the electronic medical chart contains the history data of medical treatment of the subject teeth as dental condition transition data and the server has an evaluation criterion create/update function for creating or changing the evaluation criterion data by taking the statistic on the measured value data and the history data recorded on a plurality of electronic medical charts.

11. The system of evaluating the risk of caries according to either of claims 8 through 10 wherein it is composed of a terminal having an individual evaluation criterion input function for browsing the dental condition transition data recorded on the electronic medical chart to create or modify the individual evaluation criterion data refined for each subject.

12. The system of evaluating the risk of caries according to either of claims 7 through 11 wherein the database contains the treatment guideline data giving the guideline for treatment in accordance with the evaluation criteria.

13. The system of evaluating the risk of caries according to claim 12, further comprising that the server has a treatment recommendation distribution function for distributing appropriate treatment to the individual subjects in accordance with the grade of evaluation of the risk of possible caries via any of electronic communication means such as e-mail.

14. The system of evaluating the risk of caries according to claim 12 or 13, further comprising that the treatment guideline data contains the subject treatment guideline data for the subjects and the dentist treatment guideline for the dentists.

15. The system of evaluating the risk of caries according to either of claims 7 through 14 wherein the database contains a mouth condition medicine list.

16. The system of evaluating the risk of caries according to either of claims 7 through 15 wherein the server has an inspection reservation function for accepting the evaluation of the risk of possible caries by assigning the right of use of the measuring device to the individual subjects when accessed by the subjects via their terminals via the network.

17. The system of evaluating the risk of caries according to either of claims 7 through 16 wherein the server has an evaluation distribution function for distributing grades of evaluation of the risk of possible caries to the subjects via any of electronic communication means such as e-mail.

18. The system of evaluating the risk of caries according to either of claims 7 through 17 wherein the server contains a dentist list.

19. The system of evaluating the risk of caries according to claim 18, further comprising that the server has a treatment reservation function for reserving the treatment by the dentist, who is included in the dentist list, by accessing from the subject's terminal via the network.

20. The system of evaluating the risk of caries according to either of claims 7 through 19 wherein the server has a regular inspection recommendation function for distributing the recommendation of regular inspection to the subjects depending on their grades of evaluation of the risk of possible carles via any of electronic communication means such as e-mail.

21. A program of evaluating the risk of caries having such a characteristic that it involves a step for obtaining the measured value for the chemical property of the saliva collected form the mouth of the subject, a step for referring to the evaluation criterion data in evaluating the risk of possible caries based on the measured valued for the chemical property contained in the database, and a step for outputting the obtained measured value for the chemical property in the form of the grade of evaluation of the risk of possible caries based on the evaluation criterion data, and that these steps are performed by a computer.

22. The program of evaluating the risk of caries according to claim 21, further comprising that it involves a step for storing the dental condition transition data of the subject containing the measured value for the chemical property in the database as an electronic medical chart, and that this step is performed by a computer.

23. A method of evaluating the risk of caries having such a characteristic that it measures the chemical property of the saliva collected from the mouth of the subject and outputs the measured value for chemical property in the form of the grade of evaluation of the risk of possible caries of the subject by referring to the database recording the evaluation criterion data for evaluating the risk of possible caries in accordance with the measured value, creates an electronic medical chart recording the dental condition transition data containing the measured value for the chemical property of the subject's saliva, and performs at least one of input of the measured value for the chemical property, reference to the database, and output of the grade of evaluation of the risk of possible caries through data communications via a network.
